# EUROPEAN PATENT SPECIFICATION

(11) **EP 2 004 587 B1**
(45) Date of publication and mention of the grant of the patent: **27.06.2012**
(21) Application number: 07724175.0
(22) Date of filing: 12.04.2007
(51) Int. Cl.: C07C 45/75, C07C 45/72, C07C 403/24, C07C 403/08, C07C 45/74

(54) **PROCESS FOR THE PREPARATION OF A KETONE OR AN ALDEHYDE**
VERFAHREN ZUR HERSTELLUNG EINES KETONS ODER ALDEHYDS
PROCÉDÉ DE PRÉPARATION D'UNE CÉTONE OU D'UN ALDÉHYDE

(30) Priority: 12.04.2006 EP 06007692; 30.08.2006 EP 06018054
(43) Date of publication of application: 24.12.2008
(73) Proprietor: DSM IP Assets B.V., 6411 TE Heerlen (NL)
(72) Inventor: GUMMIN, Ingolf, 79639 Grenzach-Wyhlen (DE); HAEFELE, Martin, 79713 Bad Säckingen (DE); NOESBERGER, Paul, 4127 Birsfelden (CH)
(74) Representative: Steck, Melanie
(86) International application number: PCT/EP2007/003234
(87) International publication number: WO 2007/115832

(56) References cited:
- FR-A1- 2 706 452
- US-A- 2 245 567
- US-A- 3 928 458
- PATENT ABSTRACTS OF JAPAN vol. 007, no. 114 (C-166), 18 May 1983 (1983-05-18) & JP 58 035141 A (DAICEL KK), 1 March 1983 (1983-03-01)
- RUETTIMANN A: "DIENOLETHER CONDENSATIONS - A POWERFUL TOOL IN CAROTENOID SYNTHESIS" PURE & APPLIED CHEMISTRY, PERGAMON PRESS, OXFORD, GB, vol. 71, no. 12, 1999, pages 2285-2293, XP001090911 ISSN: 0033-4545
- PFANDER: "Synthesis of optically active carotenoids: a review" PURE & APPL. CHEM., vol. 63, 1991, pages 23-33, XP002440250
- EVONIK INDUSTRIES: 'Aerolyst 3045'

## Description

The invention relates to a process for the preparation of a ketone or an aldehyde, in particular an unsaturated ketone or an unsaturated aldehyde, from a reaction mixture comprising a starting ketone and a starting aldehyde.

Such a process is generally known as an aldol condensation. An example thereof is disclosed in the Journal of Catalysis 106, pages 273-279 (1987), wherein the production of methyl vinyl ketone from formaldehyde and acetone is disclosed. The known process is a vapour-phase aldol condensation between formaldehyde and acetone using various kinds of single and binary metal oxides and heteropoly compounds as catalysts. The known process preferably uses V₂O₅-P₂O₅ (P/V atomic ratio = 1.06) and Fe₂O₃-P₂O₅ (P/Fe = 4.6) catalysts.

The known process has as a disadvantage that a significant deactivation of the catalyst is observed. In figure 4 of the said article in the Journal of Catalysis, a very significant deactivation is observed after less than 10 hours of operation. The catalyst deactivation as reported is stronger if the temperature increases from 180°C to 240°C.

It is the objective of the present invention to reduce the said disadvantage.

The objective is achieved in a process for the preparation of a ketone or an aldehyde, comprising the steps of:
a) forming a reaction mixture by bringing a mixture of a starting aldehyde and a starting ketone into the gaseous phase;
b) bringing the reaction mixture in contact with a catalyst, whereby the ketone and/or the aldehyde is/are formed,
whereby:
- at the onset of step b) the molar ratio of starting ketone to starting aldehyde in the reaction mixture lies between 9:1 and 100:1,
- step b) is executed at a temperature lying between 320°C and 380°C, and the catalyst consists of fumed silica having a purity of at least 95 % and
- does not consist of silica gel.

The advantage of the process according to the invention is that the selection of molar ratios in combination with the selection of reaction temperatures leads to a prolonged period of high catalyst activity, compared to the known process.

US-A-3,928,458 discloses that alpha, beta-unsaturated aldehydes, ketones, nitriles and esters are formed by the vapor phase condensation of saturated aldehydes, ketones, nitriles and alkyl esters of aliphatic monocarboxylic acids with formaldehyde in the presence of an unmodified silica gel catalyst, with reported conversions of formaldehyde of up to 45%.

JP 58035141 discloses a process for the preparation of a 2-cyclohexen-1one using a solid basic catalyst which can be supported on silica.

US 2 245 567 A discloses a process for the preparation of unsaturated ketones using a metal-catalyst supported on silica gel.

In step a) of the process according to the invention, a reaction mixture is formed. The reaction mixture should comprise a starting ketone and a starting aldehyde; moreover, the reaction mixture should be in the gaseous phase.

As starting ketone, in principle any known ketone or mixture of ketones is suitable, provided that the ketone of mixture of ketones is in the gaseous phase or can be brought in the gaseous phase. Ketones as such are known; preferably, a C₃ - C₁₀ aliphatic, cyclic or aromatic ketone is used. Preferred examples of ketones suitable as starting ketone include acetone, methylethylketone (MEK) and mesityloxide (4-methylpent-3-en-2-one). Within the context of the present invention, a reference to a starting ketone embraces the reference to a mixture of different starting ketones; in a preferred embodiment of the invention, the starting ketone is a mixture of ketones.

As starting aldehyde, in principle any known aldehyde or mixture of aldehydes is suitable, provided that the aldehyde or mixture of aldehydes is in the gaseous phase or can be brought in the gaseous phase. Aldehydes as such are known; preferably, a C₁ - C₁₀ aliphatic, cyclic or aromatic aldehyde is used as starting aldehyde. Preferred examples of suitable starting aldehydes include formaldehyde and acetaldehyde. Within the context of the present invention, a reference to a starting aldehyde embraces the reference to a mixture of different starting aldehydes; in a preferred embodiment of the invention, the starting aldehyde is a mixture of aldehydes.

The reaction mixture may be formed by taking a desired amount of starting materials and combining them, while preferably performing a mixing operation; if the starting materials are not in the gaseous phase, then they should be brought into the gaseous phase through means that are as such known to the skilled person, e.g. through a heating step or a decomposition step. An example of such a decomposition step is the decomposition of paraformaldehyde or trioxan to form formaldehyde. If the starting materials are already in the gaseous phase, then step a) of the present invention can already be executed through the mere combining and preferably mixing of the starting materials. The starting materials may be present as such (e.g. in pure form), or they may be present in the form of a mixture with another compound, e.g. as an aqueous solution which is quite common in for example formaldehyde. As noted above, it may be desirable to introduce a mixing step so as to homogenise the reaction mixture prior to the execution of step b).

It was found that a prolonged period of high catalyst activity can be achieved in case of a significant molar excess of the starting ketone over the starting aldehyde. According to the invention, therefore, the molar ratio between the starting ketone and the starting aldehyde is at least 9:1; more preferably, the said ratio is at least 12:1 or 15:1; in particular, the molar ratio between the starting ketone and the starting aldehyde in the reaction mixture is at least 18:1 or even 20:1; most preferably, the molar ratio between the starting ketone and the starting aldehyde in the reaction mixture is at least 24:1 or even 28:1. For practical reasons, the molar ratio between the starting ketone and the starting aldehyde in the reaction mixture is at most 100:1 or 75:1. The molar ratios as given here are applicable to the reaction mixture as it is formed, and/or at the onset of step b) as the reaction mixture comes in contact with the catalyst. It was found that an additional advantage related to a significant molar excess of the starting ketone over the starting aldehyde is that compared to the known process a higher selectivity towards compounds formed from a fusion of the starting aldehyde with the starting ketone can be achieved.

The reaction mixture may comprise additional compounds besides the starting ketone, the starting aldehyde, and water. Examples of such additional compounds are stabilizing compounds, such as methanol in case formaldehyde is used, or inert gases such as for example nitrogen, carbon dioxide, or argon. In a preferred embodiment of the invention, however, the amount of such additional compounds is limited to about 50 wt.%, 40 wt.%, 25 wt.% or even 20 or 10 or 5 wt.% of the reaction mixture. Most preferably, the reaction mixture contains essentially no additional compounds except stabilizing compounds; this has the advantage that these compounds do not need to be removed from the reaction mixture afterwards in order to isolate valuable compounds such as the ketone and/or the aldehyde. Within the context of the present invention, the terms 'contain essentially no' or 'consist essentially of' or equivalents are understood to mean that if there are any further compounds present or if other measures are taken, then these further compounds or measures have no significant influence on the functioning of the invention or on the effects as achieved by the invention.

Subsequent to the forming of the reaction mixture in step a) according to the invention, step b) is executed. In step b), the gaseous reaction mixture is brought in contact with a catalyst. Typically, and preferably, the catalyst will be a solid. The bringing in contact of the reaction mixture with the catalyst may be done by means known as such; a typical example of such a known means is putting the catalyst in a reactor and forcing the gaseous reaction mixture to flow through the reactor. It is necessary hereby that step b) is carried out at an elevated temperature lying preferably between 320°C and 380°C. More preferably, the temperature at which step b) is carried out is at least 325°C or 330°C; the temperature at which step b) is carried out is preferably at most 375°C or 370°C. It will typically not be very beneficial to carry out the process according to the invention at significantly elevated or reduced pressure, although this is possible in a very wide range between 0.005 MPa and 15 MPa.

It was found, surprisingly, that the yield of the process according to the invention can be higher than in the known process in the fumed silica as comprised in the catalyst has a high purity of at least 95, 96, 97, 98 or 99 wt.%. Preferably, the purity of the fumed silica is at least 99.1, 99.2 or 99.3 wt.%; more preferably, the purity of the fumed silica is at least 99.4, 99.5, 99.6, 99.7 or even 99.8 wt.%. Without committing to scientific explanation, it is thought that whereas in many other cases silica merely acts as and is used as a support for catalyst particles, the fumed silica itself is - surprisingly - in the process according to the invention the catalyst. It is therefore additionally preferred that the catalyst as used in the process according to the invention comprises at least 50 or 60 wt.% of high-purity fumed silica; more preferably, the catalyst as used in the process according to the invention comprises at least 75, 85 or even 90 or 95 wt.% of high-purity silica; most preferably, the catalyst as used in the process according to the invention consists essentially or even only of high-purity fumed silica. The fumed silica, however, should not consist of silica gel. It was found that the use of silica gel can lead to conversions and yields that are unsatisfactory.

The catalyst as used in the process according to the invention is free of silica gel.

It is preferred that if there are any compounds comprised in the catalyst, then these are essentially devoid of Al₂O₃, ZrO₂, P₂O₅, V₂O₅, Ce₂O₃, TiO₂, and Na₂O; it was found that the said compounds are among the compounds that can have a negative influence on the yield and/or selectivity of the ketone- or aldehyde-forming reaction.

According to the invention, the silica as comprised in the solid catalyst consists of fumed silica. As is known, fumed silica - also referred to as pyrogenic silica - is different from silica gel. Whereas silica gel is typically prepared by the neutralization of aqueous alkali metal silicate with acid, fumed silica is typically prepared by vaporizing a silicon tetrahalide such as silicon tetrachloride, mixing it with dry air and hydrogen, then feeding the mixture to a burner and hydrolyzing it. The differences between silica gel and fumed silica are further elucidated in literature, such as in the Silica chapter of Ullmann's Encyclopedia of Industrial (Electronic version) as posted online on 15.09.2000 with DOI 10.1002/14356007.a23 583. As is known, fumed silica preferably consists of clusters of primary non-porous particles, whereby the cluster structure creates pores. This structure results in the fumed silica having a high specific surface area, although the specific surface area of fumed silica is on average lower than that of silica gel; preferably, this specific surface area of the fumed silica lies between 25 and 500 m²/g, more preferably between 50 and 450 m²/g. It is furthermore preferred that the fumed silica has an amount of at most 20% micropores or even at most 15% or 10% or essentially no micropores at all. Preferably, thus, the catalyst is essentially free of micropores. Micropores are defined as pores having a diameter of 5 nm or less. The low amount or even absence of micropores is advantageous as micropores are difficult to access for the compounds in the reaction mixture, or even not accessible at all. It is furthermore preferred that the low amount of micropores is combined with an average pore size lying between 5 and 100 nm, more preferably between 6 nm and 50 nm. Most preferably, the amount of pores having a diameter of more than 100 nm is at most 10%, 5% or even at most 1%; as a consequence of this, the pore size distribution will be relatively narrow. The low amount or even absence of micropores can contribute to the catalyst having a specific surface area that lies lower than values of 500 or even 600 m²/g. Preferably, therefore, the specific surface area of the catalyst as used in the process of the invention is at most 400, 350, 300 or even at most 250 or 200 m²/g. This has - in particular in combination with a low amount or even absence of micropores - the advantage that maximum use can be made of essentially the whole of the specific surface area of the catalyst, thus contributing to a high degree of conversion. In order to contribute to a high degree of conversion even in a limited reaction space, it is preferred that the silica catalyst has a specific surface area of at least 25, 50, 75 or 100 m²/g.

The contact of the reaction mixture with the catalyst can lead to several types of reaction mechanisms, which are embraced within the context of the present invention. The reactions may take place on the surface of the catalyst or they may take place in the gaseous phase, e.g. by a reactive intermediate compound that was formed on the catalyst and then detached from it. The reactions taking place at the catalyst may start either from a ketone, from an aldehyde, or from both.

The nature of the starting ketone and the starting aldehyde will co-determine which ketone or mixture of ketones will be formed. As is the case in the known process, the presence of acetone as the starting ketone and formaldehyde as the starting aldehyde can lead to the formation of a.o. methylvinylketone (MVK), a know compound having formula (I):

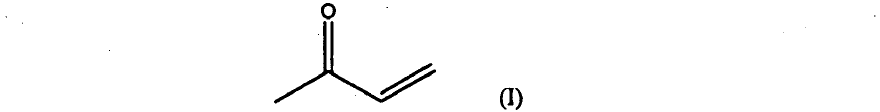

Since MVK is a compound having commercial importance, a.o. as intermediate compound in the synthesis of Vitamin A, astaxanthin and zeaxanthin, the reaction mixture in a preferred embodiment of the process according to the invention comprises upon its formation both a starting ketone and a starting aldehyde, whereby acetone is primarily or even essentially the starting ketone and formaldehyde is primarily or even essentially the starting aldehyde.

As indicated above, the formation of a reaction mixture comprising formaldehyde as starting aldehyde may for practical reasons involve the use of an aqueous solution of formaldehyde; such solutions, e.g. comprising 37 wt.% formaldehyde in water, are known. Generally speaking, the person skilled in the art would expect that the presence of water has a negative influence on the overall yield of the reaction since water is a by-product in many aldol condensation reactions such as MVK formation from formaldehyde and acetone, and thus could lead to an equilibrium shift away from end product formation. It was surprisingly found, however, that the presence of water in the reaction mixture in step a) or at the onset of step b) does not have a negative effect; by contrast, a positive effect on the yield of MVK was observed. In a preferred embodiment of the invention, therefore, the weight ratio of formaldehyde to water in the reaction mixture is in step a) or at the onset of step b) at most 60:40, 50:50 or 40:60, preferably at most 30:70 or 25:75, most preferably at most 20:80. The reaction mixture as a whole preferably comprises - at the onset of the execution of step b) - at least 1 wt.% of water, more preferably at least 5, 10 or even 15 wt.%; the reaction mixture as a whole preferably comprises - at the onset of the execution of step b) - at most 50 wt.% water, more preferably at most 40, 30 or 20 wt.%.

The steps a) and b) according to the invention may be carried out in batch operation or in continuous operation. In any case, it is preferred that the residence time of the reaction mixture in step b), in particular the contact time between catalyst and reaction mixture, is chosen such that an adequate reaction progression is combined with an economical mode of operation. In achieving this, it is helpful to consider the widely used residence time parameter W/F; this parameter is defined as the amount of catalyst present (in grams) divided by the flow of the reaction mixture (in total amount of moles of all compounds in the reaction mixture per hour). In the process according to the invention, it is preferred that W/F lies between 1 and 1000 g.h/mol, more preferably between 10 and 750 g.h/mol, in particular between 20 and 500 g.h/mol, and most preferably between 40 and 150 g.h/mol.

Aside from achieving the objective of reducing catalyst deactivation, the process according to the invention can also be carried out such that another objective, being a high conversion of the starting compound(s), can be achieved in step b). It is hereby noted that the term conversion as meant herein should be evaluated on that starting compound or that mixture of starting compounds that is understoichiometrically present, i.e. the starting aldehyde. The process according to the invention can preferably be carried out in such a way that the conversion in step b) of at least the understoichiometrically present starting compound(s) is at least 50%. Preferably, the process according to the invention is carried out such that the conversion in step b) of at least the understoichiometrically present starting compound(s) is at least 60%, 70% or even at least 80%. The said conversions may favourably be achieved by selecting a high purity silica catalyst, whereby the silica preferably comprises or consists essentially of fumed silica, in combination with a favourable combinations of reaction conditions in step b). It was found that important reaction conditions in this respect are the temperature and the residence parameter W/F; furthermore, it was found that the structure of the catalyst can be beneficial in this regard. The conditions as far as the temperature is concerned have already been described herein with the aim of reducing catalyst deactivation; the same temperature conditions contribute to achieving a high degree of conversion. Also, as described above, it is preferred that the residence time parameter W/F lies between 1 and 1000 g.h/mol, more preferably between 10 and 750 g.h/mol, in particular between 20 and 500 g.h/mol, and most preferably between 40 and 150 g.h/mol. A specific choice of the composition or structure of the silica catalyst can further contribute to achieving an enhanced conversion, e.g by selecting a very high purity and/or by selecting fumed silica and/or by ensuring that the fumed silica has a low amount of micropores. The person skilled in the art will be able to find through routine optimisations within the ranges as given the conditions that enable him to achieve a conversion of at least 50%, preferably at least 60, 70 or 80%. The said routine optimisations will also yield those combinations of catalyst type and reaction conditions that combine a high conversion of the starting compound(s) with a high selectivity towards the desired ketone and/or aldehyde to be formed. As described earlier, a high selectivity may be further enhanced by other measures such as the presence of water in the reaction mixture.

Since the reaction mixture comprises - at least in step a) and at the onset of step b) - an excess of the starting ketone, the situation can arise that the reaction mixture comprises a high amount of acetone as starting ketone subsequent to the execution of step b). It is then typically desirable to recover the starting ketone and/or the starting aldehyde from the reaction mixture. The invention therefore further also relates to a process comprising after step b) the steps of:
c) feeding the reaction mixture to a rectification apparatus;
d) separating a top stream comprising the starting ketone and/or the starting aldehyde from the reaction mixture and removing it from the rectification apparatus;
e) optionally recycling at least a portion of the top stream to step a) or step b).

In step c) according to the invention, the reaction mixture is fed to a rectification apparatus. Rectification apparatuses are as such known; an example of such an apparatus is a distillation column. It is preferred to feed the reaction mixture to the rectification apparatus with the reaction mixture being in the gaseous phase, preferably without having undergone phase changes subsequent to the completion of step b). This has, in combination with the subsequent steps d) and e), the advantage that hardly any energy related to changing of phase is lost in the recovery of the starting ketone and/or the starting aldehyde. In the rectification apparatus, step d) according to the invention takes place: a - preferably gaseous - stream comprising the starting ketone and/or the starting aldehyde is separated from the reaction mixture, and subsequently removed from the rectification apparatus. Since this stream will often be removed from the upper half of the apparatus, it is hereby named the top stream. The forming of the top stream by separating it from the reaction mixture may be achieved by means known as such to the skilled person, such as distillation techniques. Also the removal of the top stream from the rectification apparatus may be achieved by means known as such to the skilled person, such as pumping or pressure reduction.

The top stream may now, after having been removed from the rectification apparatus, be advantageously re-used in the process according to the invention. The invention therefore preferably comprises - subsequent to step d) - a step e), in which at least a portion of the top stream is recycled to step a) or step b). The said portion is preferably at least 25 wt.% of the top stream, more preferably at least 50 wt.% or even at least 75 wt.%. The separating off of a portion from the top stream and the subsequent recycling may be achieved by means as such known to the skilled person.

It may be additionally advantageous to separate a second portion off from the top stream, condense this second portion and feed it back to the rectification apparatus as a reflux stream. As is know to the skilled person, such reflux streams are often beneficial or even necessary in achieving a satisfactory composition of the top stream and in achieving a satisfactory energy efficiency of operation of the rectification apparatus.

The starting ketone and/or starting aldehyde as comprised in the top stream can - as described above - be beneficially recycled to step a) or b) of the process according to the invention so as to aid in forming the reaction mixture. Depending on the nature of the top stream, it may be necessary to implement one or more purification steps on the top stream so as to remove any unwanted compounds.

With the top stream separated off from the reaction mixture, the reaction mixture may be removed from the rectification apparatus as well - preferably after the reaction mixture has been brought into liquid form. Depending on the circumstances, subsequent steps may be desirable in order to isolate the ketone and/or the aldehyde from the reaction mixture. Examples of such steps are rectification, filtration, extraction, crystallisation, and the like.

In case the reaction mixture comprises acetone as starting ketone and formaldehyde as starting aldehyde, then - as indicated above - MVK can be prepared. When a gaseous reaction mixture comprising MVK is then according to step c) fed to a rectification apparatus and brought into liquid form, it was found that it is important to ensure that undesirable subsequent reactions of MVK such as polymerisation are minimised. It is therefore a further object of the invention to provide a method for reducing or even preventing the said subsequent reactions of MVK. The said method comprises the step of mixing an MVK-stabilizing agent with MVK when the MVK is transferred from the gaseous phase into the liquid phase or immediately thereafter. The MVK-stabilizing agent preferably comprises water; more preferably, the MVK-stabilizing agent comprises a mixture of water with an acid. Preferably, the acid is acetic acid. In particular, the MVK-stabilizing agent comprises besides water and an acid such as acetic acid also hydroquinone. If the MVK-stabilizing agent comprises hydroquinone, then it is preferred that it also comprises acetonitrile. In a preferred embodiment of the invention, the MVK-stabilizing agent has the following composition:
- between 30 and 95 wt.% acetic acid, preferably between 50 and 90 wt.%;
- between 1 and 40 wt.% water, preferable between 10 and 30 wt.%;
- between 0 and 15 wt.% hydroquinone, preferably between 0.1 and 10 wt.%;
- between 0 and 15 wt.% acetonitrile, preferably between 0.1 and 10 wt.%,
whereby the sum of these four compounds adds to 100%.

The amount of MVK-stabilizing agent as added to the reaction mixture, or to the MVK itself after having been further purified, may vary between wide limits and lies preferably between 0.1 and 10 wt.% relative to the amount of MVK.

The minimisation of undesirable subsequent reactions of ketones such as MVK or of aldehydes may also be supported by choosing favourable process conditions. It was found that the speed of said undesirable subsequent reactions is reduced with reducing temperature; this may be achieved by for example carrying out the process in the rectification apparatus at reduced pressure. Furthermore, it was found that the absence of light also contributes to the control of undesirable subsequent reactions.

The preparation of MVK constitutes one example of the industrial applicability of the process according to the invention. Another example of an unsaturated ketone that may be formed according to the invention is the preparation of 3-methylcyclohexenon from a reaction mixture comprising formaldehyde as starting aldehyde and mesityloxide as starting ketone. The compound 3-methylcyclohexenon is as such known and has formula (II):

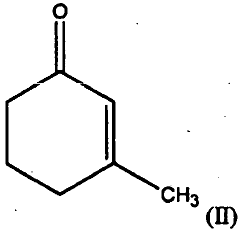

Generally speaking, it will be advantageous to choose the starting material(s) such that the formation of those ketones and/or aldehydes is targeted that permit handling in the gaseous phase at the prevailing temperatures in step b). A quick or even immediate phase transfer of the ketones and/or aldehydes as formed into the liquid or solid phase would be more likely to cause processing problems and is thus less preferred.

The invention will be illustrated by the following examples and comparative experiments, without being limited thereto.

### Examples 1 - 6

The preparation of MVK according to steps a) and b) was executed in a unit comprising a vaporiser and a reactor. The vaporiser comprised two sections: in the first section acetone as starting ketone was vaporised by means of heating; in the second section a 37 wt.% aqueous solution of the starting aldehyde formaldehyde was vaporised by means of spraying it into the gaseous acetone stream and heating; hereby the reaction mixture was formed. The heating was done such that the reaction mixture had a temperature equalling the reactor temperature. The reaction mixture was then fed to a reactor containing a catalyst. The temperature in the reactor was as indicated in the table. The catalyst type is as given in the table below. As the reaction mixture exited the reactor, a sample was taken and analysed. Each sample was taken after the catalyst had been in operation for a number of hours as indicated in the table. The residence time of the reaction mixture in the reactor is given in the table by means of the residence parameter W/F.

From the data in the table, it is evident that yields in MVK formation are higher than as reported in the prior art and, furthermore, that these high yields are achieved over much longer periods of time that the up to 8 hours as reported in the Journal of Catalysis. Additionally, it can be seen that an increase of the molar ratio between acetone and formaldehyde - within the range according to the invention - is beneficial, as is a raise in temperature.

| Example / Comparative Experiment | Catalyst | Residence time in reactor W/F (g.h/mol) | Molar ratio acetone / formaldehyde | Reactor Temperature (°C) | Catalyst Operational Hours | Conversion of Formaldehyde (%) | Yield of MVK (%) |
|---|---|---|---|---|---|---|---|
| 1-I | Aerolyst^{®} 3045 | 55 | 16 | 350 | 10 | 89 | 71 |
| 1-II | Aerolyst^{®} 3045 | 55 | 16 | 350 | 50 | 82 | 68 |
| 2-I | Aerolyst^{®} 3045 | 55 | 24 | 350 | 10 | 97 | 77 |
| 2-II | Aerolyst^{®} 3045 | 55 | 24 | 350 | 50 | 89 | 74 |
| 3-I | Aerolyst^{®} 3045 | 55 | 28 | 350 | 10 | 98 | 81 |
| 3-II | Aerolyst^{®} 3045 | 55 | 28 | 350 | 50 | 95 | 80 |
| 4-I | Aerolyst^{®} 3045 | 72 | 23 | 330 | 20 | 95 | 79 |
| 4-II | Aerolyst^{®} 3045 | 72 | 23 | 330 | 50 | 88 | 70 |
| 5-I | Aerolyst^{®} 3045 | 72 | 23 | 350 | 20 | 99 | 79 |
| 5-II | Aerolyst^{®} 3045 | 72 | 23 | 350 | 50 | 96 | 76 |
| 6-I | Aerolyst^{®} 3045 | 72 | 23 | 370 | 20 | 99 | 79 |
| 6-II | Aerolyst^{®} 3045 | 72 | 23 | 370 | 50 | 98 | 79 |

## Claims

1. Process for the preparation of a ketone or an aldehyde, comprising the steps of:
a) forming a reaction mixture by bringing a mixture of a starting aldehyde and a starting ketone into the gaseous phase;
b) bringing the reaction mixture in contact with a catalyst, whereby the ketone and/or the aldehyde is/are formed,
whereby:
• at the onset of step b) the molar ratio of starting ketone to starting aldehyde in the reaction mixture lies between 9:1 and 100:1,
• step b) is executed at a temperature lying between 320°C and 380°C, and **characterized in that**
the catalyst consists of fumed silica having a purity of at least 95 % and does not consist of silica gel.

2. Process according to claim 1, wherein the silica has a purity of at least 98 %.

3. Process according to claim 1 or 2, wherein the starting ketone comprises acetone and the starting aldehyde comprises formaldehyde.

4. Process according to any of the claims 1 to 3, wherein the reaction mixture comprises, at least at the moment it is brought in contact with the catalyst, between 1 and 50 wt.% water.

5. Process according to claim 4, wherein the reaction mixture comprises, at least at the moment it is brought in contact with the catalyst, less than 10 wt.% of compounds other than compounds from the group consisting of aldehydes, ketones, stabilizers, and water.

6. Process according to clam 1 , further comprising after step b) the steps of:
c) feeding the reaction mixture in the gaseous phase to a rectification apparatus;
d) separating a top stream comprising the starting ketone and/or the starting aldehyde from the reaction mixture and removing it from the rectification apparatus;
e) optionally recycling at least a portion of the top stream to step a) or step b).

7. Process according to claim 6, wherein the starting ketone comprises acetone and the starting aldehyde comprises formaldehyde, wherein the ketone comprises methylvinylketone (MVK), said MVK being transferred into the liquid phase, whereby an MVK-stabilizing compound is mixed with MVK at the moment MVK is liquefied or shortly thereafter.

8. Process according to claim 7, wherein the MVK-stabilizing compound is a mixture comprising at least one of water, acetic acid, hydrochinone and acetonitrile.

9. Process for the preparation of vitamin A, comprising the process according to any one of claims 1 - 8.

10. Process for the preparation of astaxanthin, comprising the process according to any one of claims 1 - 8.

11. Process for the preparation of zeaxanthin, comprising the process according to any one of claims 1 - 8.

## Patentansprüche

1. Verfahren zur Herstellung eines Ketons oder eines Aldehyds, bei dem man:
a) eine Reaktionsmischung bildet, indem man eine Mischung eines Ausgangsaldehyds und eines Ausgangsketons in die Gasphase bringt;
b) die Reaktionsmischung mit einem Katalysator in Kontakt bringt, wobei das Keton und/oder der Aldehyd gebildet werden/wird,
wobei:
• zu Beginn von Schritt b) das Molverhältnis von Ausgangsketon zu Ausgangsaldehyd in der Reaktionsmischung zwischen 9:1 und 100:1 liegt,
• Schritt b) bei einer Temperatur zwischen 320°C und 380°C durchgeführt wird, und
**dadurch gekennzeichnet, dass** der Katalysator aus pyrogenem Siliciumdioxid mit einer Reinheit von mindestens 95% besteht und nicht aus Kieselgel besteht.

2. Verfahren nach Anspruch 1, bei dem das Siliciumdioxid eine Reinheit von mindestens 98% aufweist.

3. Verfahren nach Anspruch 1 oder 2, bei dem das Ausgangsketon Aceton umfasst und der Ausgangsaldehyd Formaldeyhd umfasst.

4. Verfahren nach einem der Ansprüche 1 bis 3, bei dem die Reaktionsmischung zumindest in dem Moment, in dem sie mit dem Katalysator in Kontakt gebracht wird, zwischen 1 und 50 Gew.-% Wasser umfasst.

5. Verfahren nach Anspruch 4, bei dem die Reaktionsmischung zumindest in dem Moment, in dem sie mit dem Katalysator in Kontakt gebracht wird, weniger als 10 Gew.-% andere Verbindungen als Verbindungen aus der Gruppe bestehend aus Aldehyden, Ketonen, Stabilisatoren und Wasser umfasst.

6. Verfahren nach Anspruch 1, bei dem man ferner nach Schritt b):
c) die Reaktionsmischung in der Gasphase einer Rektifikationsapparatur zuführt;
d) aus der Reaktionsmischung einen Kopfstrom, der das Ausgangsketon und/oder den Ausgangsaldehyd umfasst, abtrennt und aus der Rektifikationsapparatur entfernt;
e) gegebenenfalls mindestens einen Teil des Kopfstroms zu Schritt a) oder Schritt b) rezykliert.

7. Verfahren nach Anspruch 6, bei dem das Ausgangsketon Aceton umfasst und der Ausgangsaldehyd Formaldeyhd umfasst, wobei das Keton Methylvinylketon (MVK) umfasst, wobei das MVK in die Flüssigphase überführt wird, wobei in dem Moment, in dem MVK verflüssigt wird, oder kurz danach eine MVKstabilisierende Verbindung mit MVK gemischt wird.

8. Verfahren nach Anspruch 7, wobei es sich bei der MVK-stabilisierenden Verbindung um eine Mischung, die Wasser, Essigsäure, Hydrochinon und/oder Acetonitril umfasst, handelt.

9. Verfahren zur Herstellung von Vitamin A, das das Verfahren nach einem der Ansprüche 1-8 umfasst.

10. Verfahren zur Herstellung von Astaxanthin, das das Verfahren nach einem der Ansprüche 1-8 umfasst.

11. Verfahren zur Herstellung von Zeaxanthin, das das Verfahren nach einem der Ansprüche 1-8 umfasst.

## Revendications

1. Procédé pour la préparation d'une cétone ou un aldéhyde, comprenant les étapes consistant à :
a) former un mélange de réaction en amenant un mélange d'un aldéhyde de départ et d'une cétone de départ dans la phase gazeuse ;
b) amener le mélange de réaction en contact avec un catalyseur, de telle manière que la cétone et/ou l' aldéhyde soi(en)t formé(s),
dans lequel :
• au début de l'étape b) le rapport molaire de la cétone de départ à l'aldéhyde de départ dans le mélange de réaction est situé entre 9:1 et 100:1,
• l'étape b) est exécutée à une température située entre 320 °C et 380 °C, et
**caractérisé en ce que**
le catalyseur est constitué de fumée de silice ayant une pureté d'au moins 95 % et n'est pas constitué de gel de silice.

2. Procédé selon la revendication 1, dans lequel la silice a une pureté d'au moins 98 %.

3. Procédé selon la revendication 1 ou 2, dans lequel la cétone de départ comprend de l'acétone et l'aldéhyde de départ comprend du formaldéhyde.

4. Procédé selon l'une quelconque des revendications 1 à 3, dans lequel le mélange de réaction comprend, au moins au moment auquel il est mis en contact avec le catalyseur, entre 1 et 50 % en poids d' eau.

5. Procédé selon la revendication 4, dans lequel le mélange de réaction comprend, au moins au moment auquel il est mis en contact avec le catalyseur, moins de 10 % en poids de composés autres que des composés du groupe constitué d'aldéhydes, de cétones, de stabilisants, et d'eau.

6. Procédé selon la revendication 1, comprenant en outre après l'étape b) les étapes consistant à :
c) charger le mélange de réaction dans la phase gazeuse dans un appareil de rectification ;
d) séparer un flux supérieur comprenant la cétone de départ et/ou l'aldéhyde de départ du mélange de réaction et retirer celui-ci de l'appareil de rectification ;
e) facultativement recycler au moins une partie du flux supérieur dans l'étape a) ou l'étape b).

7. Procédé selon la revendication 6, dans lequel la cétone de départ comprend de l'acétone et l'aldéhyde de départ comprend du formaldéhyde, dans lequel la cétone comprend de la méthylvinylcétone (MVK), ledit MVK étant transféré dans la phase liquide, de telle manière qu'un composé stabilisant MVK soit mélangé avec MVK au moment auquel MVK est liquéfié ou peu après.

8. Procédé selon la revendication 7, dans lequel le composé stabilisant MVK est un mélange comprenant au moins l'un de l'eau, l'acide acétique, l'hydrochinone et l'acétonitrile.

9. Procédé pour la préparation de vitamine A, comprenant le procédé selon l'une quelconque des revendications 1 à 8.

10. Procédé pour la préparation d'astaxanthine, comprenant le procédé selon l'une quelconque des revendications 1 à 8.

11. Procédé pour la préparation de zéaxanthine, comprenant le procédé selon l'une quelconque des revendications 1 à 8.
